# EUROPÄISCHE PATENTSCHRIFT

(11) **EP 0 824 518 B1**
(45) Veröffentlichungstag und Bekanntmachung des Hinweises auf die Patenterteilung: **27.06.2001**
(21) Anmeldenummer: 96913530.0
(22) Anmeldetag: 26.04.1996
(51) Int. Cl.: C07D 213/55, A01N 43/40, C07C 69/618, A01N 37/10, C07D 239/26, A01N 43/54, C07D 239/34, C07D 239/30, C07D 403/12, C07D 413/12, A01N 43/84

(54) **(HET)ARYLOXY-, -THIO-, AMINOCROTONATE, VERFAHREN ZU IHRER HERSTELLUNG UND IHRE VERWENDUNG ZUR BEKÄMPFUNG VON TIERISCHEN SCHÄDLIN GEN UND SCHADPILZEN**
(HET)ARYLOXY-, -THIO-, AMINOCROTONATES, METHODS OF PREPARING THEM AND THEIR USE AS INSECTICIDES AND FUNGICIDES
(HET)ARYLOXY, THIO, AMINOCROTONATES, LEUR PROCEDE DE FABRICATION ET LEUR UTILISATION COMME INSECTICIDES ET COMME FONGICIDES

(30) Priorität: 09.05.1995 DE 19516844
(43) Veröffentlichungstag der Anmeldung: 25.02.1998
(73) Patentinhaber: BASF AKTIENGESELLSCHAFT, 67056 Ludwigshafen (DE)
(72) Erfinder: GROTE, Thomas, D-37077 Göttingen (DE); KIRSTGEN, Reinhard, D-67434 Neustadt (DE); MÜLLER, Bernd, D-67227 Frankenthal (DE); SAUTER, Hubert, D-68167 Mannheim (DE); HARREUS, Albrecht, D-67063 Ludwigshafen (DE); KÖNIG, Hartmann, D-69115 Heidelberg (DE); AMMERMANN, Eberhard, D-64646 Heppenheim (DE); LORENZ, Gisela, D-67434 Hambach (DE); STRATHMANN, Siegfried, D-67117 Limburgerhof (DE); RÖHL, Franz, D-67105 Schifferstadt (DE)
(86) Internationale Anmeldenummer: EP9601754
(87) Internationale Veröffentlichungsnummer: WO9635669

(56) Entgegenhaltungen:
- EP-A- 0 212 859
- EP-A- 0 348 766
- EP-A- 0 383 117
- EP-A- 0 409 369
- EP-A- 0 471 261
- EP-A- 0 584 625
- GB-A- 2 238 308

## Beschreibung

Die vorliegende Erfindung betrifft (Het)aryloxy-, -thio- und-aminocrotonate der Formel I 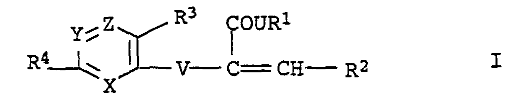 in der die Substituenten die folgenden Bedeutung haben:
- U: Sauerstoff (-O-), Schwefel (-S-) oder Amino (-NH-);
- V: Sauerstoff (-O-), Schwefel (-S-), Amino (-NH-) oder C₁-C₁₀-Alkylamino [-N(C₁-C₁₀-Alkyl)-];
- X, Y, Z: unabhängig voneinander =N- oder =CR³-;
- R¹, R²: unabhängig voneinander C₁-C₄-Alkyl;
- R³: Wasserstoff, Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
- R⁴: eine Gruppe A¹-Y¹ oder eine Gruppe A²-W^{a}-N=CR^{a}-;
- A¹: C₁-C₁₀-Alkyl, C₂-C₁₀-Alkenyl oder C₂-C₁₀-Alkinyl,
wobei diese Gruppen partiell oder vollständig halogeniert sein können und einen der folgenden Reste tragen können: unsubstituiertes Phenyl oder durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio substituiertes Phenyl,
ein- bis dreikerniges aromatisches Ringsystem enthaltend 6-14 Kohlenstoffringglieder, 5-gliedriges Hetaryl enthaltend ein bis vier Stickstoffatome oder ein bis drei Stickstoffatome und ein Schwefel oder Sauerstoffatom, oder 6-gliedriges Hetaryl enthaltend ein bis drei Stickstoffatome,
wobei die (hetero)aromatischen Gruppen durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio substituiert sein und/oder einen oder zwei der folgenden Reste tragen können: Formyl oder CRⁱⁱⁱ=NOR^{iv},
wobei Rⁱⁱⁱ Wasserstoff oder C₁-C₄-Alkyl und R^{iv} C₁-C₄-Alkyl und Phenyl-C₁-C₄-alkyl bedeuten,
oder Phenyl, Pyridyl, Pyrimidyl oder Triazinyl,
wobei diese Gruppen durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio substituiert sein können;
- R^{a}: Wasserstoff, C₁-C₁₀-Alkyl oder C₁-C₁₀-Halogenalkyl;
- W^{a}: Sauerstoff, Amino oder C₁-C₁₀-Alkylamino;
- A²: Wasserstoff,
C₁-C₁₀-Alkyl, C₂-C₁₀-Alkenyl oder C₂-C₁₀-Alkinyl,
wobei diese Gruppen partiell oder vollständig halogeniert sein können und einen der folgenden Reste tragen können:
unsubstituiertes Phenyl oder durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino oder C₁-C₄-Alkylthio substituiertes Phenyl.

Außerdem betrifft die Erfindung Verfahren zur Herstellung dieser Verbindungen, sie enthaltende Mittel und ihre Verwendung zur Bekämpfung von tierischen Schädlingen und Schadpilzen.

Aus der Literatur sind (Het)aryloxy-, -thio- und -amino-β-alkoxyacrylate mit fungizider Wirkung bekannt (EP-A 212 859; EP-A 383 117; EP-A 471 261; EP-A 503 436; EP-A 584 625). Außerdem werden in der EP-A 409 369 Hetaryloxy- und -thiocrotonate mit herbizider, wachstumsregulierender und fungizider Wirkung beschrieben, bei denen der Hetarylring entweder für einen Pyrimidin-2-ylring oder einen 1,3,5-Triazin-2-ylring steht.

Der vorliegenden Erfindung lagen Verbindungen mit verbesserten Eigenschaften als Aufgabe zugrunde.

Demgemäß wurden die eingangs definierten Verbindungen I gefunden. Außerdem wurden Verfahren zur Herstellung dieser Verbindungen, sie enthaltende Mittel und ihre Verwendung zur Bekämpfung von tierischen Schädlingen und Schadpilzen gefunden.

Die Verbindungen I können im allgemeinen nach den in der zitierten Literatur beschriebenen Verfahren hergestellt werden.

Bevorzugt erhält man die Verbindungen I, in denen V Sauerstoff oder Schwefel bedeutet dadurch, daß man einen entsprechenden Alkohol bzw. ein entsprechendes Thiol der Formel IIa in an sich bekannter Weise (J. Chem. Soc. 1963, 4210) in Gegenwart einer Base mit einem Crotonat der Formel III umsetzt.

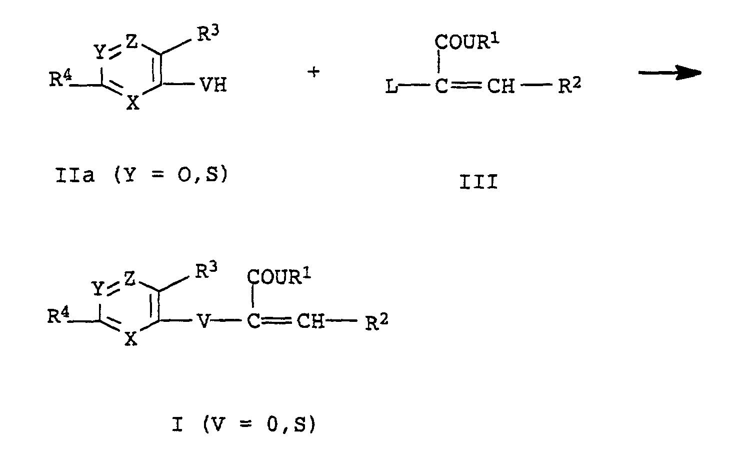

L in der Formel III steht für eine nucleophil austauschbare Abgangsgruppe wie Halogen (z.B. Chlor, Brom und Jod) oder ein Sulfonat (z.B. Trifluormethylsulfonat, Phenylsulfonat und p-Methylphenylsulphonat).

Diese Umsetzung erfolgt üblicherweise bei Temperaturen von 0°C bis 200°C, vorzugsweise 0°C bis 150°C, insbesondere 10°C bis 100°C, in einem inerten Lösungs- oder Verdünnungsmittel.

Geeignete Lösungs- oder Verdünnungsmittel sind aliphatische Kohlenwasserstoffe wie Pentan, Hexan, Cyclohexan und Petrolether,
aromatische Kohlenwasserstoffe wie Toluol, o-, m- und p-Xylol, halogenierte Kohlenwasserstoffe wie Methylenchlorid, Chloroform und Chlorbenzol,
Ether wie Diethylether, Diisopropylether, tert.-Butylmethylether, Dioxan, Anisol und Tetrahydrofuran,
Nitrile wie Acetonitril und Propionitril,
Ketone wie Aceton, Methylethylketon, Diethylketon und tert.-Butylmethylketon,
Alkohole wie Methanol, Ethanol, n-Propanol, Isopropanol, n-Butanol und tert.-Butanol
sowie Dimethylsulfoxid und Dimethylformamid,
besonders bevorzugt Acetonitril, Dimethylformamid, Dimethylsulfoxid und 1,3-Dimethyltetrahydro-2(IH)-pyrimidinon (DMPU). Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Kaliumcarbonat, Kaliumhydroxid und Natriumhydrogencarbonat.

Die Basen werden im allgemeinen äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, II in einem Über- oder Unterschuß bezogen auf III einzusetzen.

Die für die Herstellung der Verbindungen I benötigten Ausgangsstoffe der Formel IIa sind aus der Literatur bekannt oder können gemäß der im Zusammenhang mit der Beschreibung der Herstellung der einzelnen Gruppen R⁴ zitierten Literatur synthetisiert werden.

Die für die Herstellung weiterhin benötigten Ausgangsstoffe der Formel III sind ebenfalls aus der Literatur bekannt [J. Org. Chem. 44, 2608 (1979); J. Chem. Soc. PTI 21, 2651 (1993)] oder können gemäß dieser Literatur erhalten werden.

Verbindungen I, in denen V für Amino oder Alkylamino steht, erhält man bevorzugt dadurch, daß man ein entsprechendes Amin der Formel IIb in an sich bekannter Weise (Synthesis 1975, 512) in Gegenwart von Protonensäuren mit einer α-Ketocarbonylverbindung der Formel IV umsetzt.

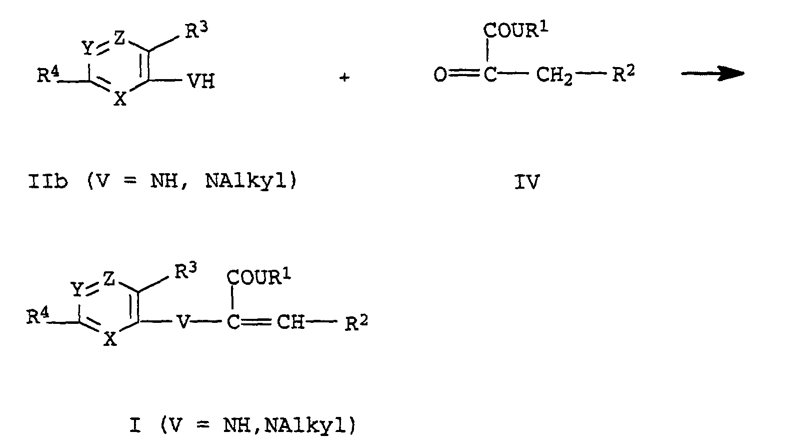

Diese Umsetzung erfolgt üblicherweise bei Temperaturen von 0°C bis 200°C, vorzugsweise 0°C bis 150°C, insbesondere 10°C bis 150°C, in einem inerten Lösungs- oder Verdünnungsmittel.

Geeignete Lösungs- oder Verdünnungsmittel sind aliphatische Kohlenwasserstoffe wie Pentan, Hexan, Cyclohexan und Petrolether,aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol.

Es können auch Gemische der genannten Lösungsmittel verwendet werden.

Als Protonensäuren finden
anorganische Säuren wie Fluorwasserstoffsäure, Salzsäure, Bromwasserstoffsäure, Schwefelsäure und Perchlorsäure,
sowie organische Säuren wie Ameisensäure, Essigsäure, Propionsäure, Oxalsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, II in einem Über- oder Unterschuß bezogen auf IV einzusetzen.

Die für die Herstellung der Verbindungen I benötigten Ausgangsstoffe der Formel IIb sind aus der Literatur bekannt oder können gemäß der im Zusammenhang mit der Beschreibung der Herstellung der einzelnen Gruppen R⁴ zitierten Literatur synthetisiert werden.

Die für die Herstellung weiterhin benötigten Ausgangsstoffe der Formel IV sind ebenfalls aus der Literatur bekannt
[Helv. Chim. Acta 30, 1372 (1947); J. Org. Chem. 43, 4245 (1978); Biochemistry 10, 2669 (1971)] oder können aus dieser Literatur erhalten werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Synthese der Gruppe R⁴ kann unabhängig von den vorstehend beschriebenen Kupplungen erfolgen und richtet sich nach der Natur der Gruppe R⁴. Zur besseren Übersichtlichkeit ist die -V-C(COUR¹)=CH-R² oder deren Vorstufe in den folgenden Reaktionsschemata verkürzt als R# dargestellt.

Üblicherweise geht man bei der Synthese von Verbindungen I, bei denen R⁴ über ein Kohlenstoffatom gebunden ist, von der entsprechenden Methyl- bzw. Alkylverbindung aus (I.1) und halogeniert diese Verbindung I.1 zunächst zum entsprechenden Benzylhalogenid I.2 [vgl. Angew. Chem 71, 349 (1959)].

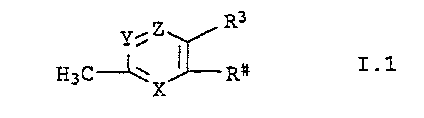

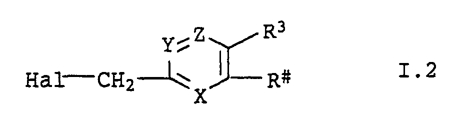

Diese Halogenierung erfolgt üblicherweise bei Temperaturen von 0°C bis 100°C, vorzugsweise 20°C bis 80°C in einem inerten Lösungsmittel entweder in Gegenwart eines Radikalstarters (z.B. Dibenzoylperoxid oder Azobisisobutyronitril oder unter UV-Bestrahlung, z.B. mit einer Quecksilberdampflampe.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, sowie Ether wie Diethylether, Diisopropylether, tert.-Butylmethylether, Dioxan, Anisol und Tetrahydrofuran, besonders bevorzugt Cyclohexan, Methylenchlorid und Tetrachlorkohlenstoff. Es können auch Gemische der genannten Lösungsmittel verwendet werden.

Als Halogenierungsmittel dienen beispielsweise elementare Halogene (z.B. Cl₂, Br₂, I₂), N-Brom-Succinimid, N-Chlorsuccinimid oder Dibromdimethylhydrantoin. Die Halogenierungsmittel werden im allgemeinen äquimolar, im Überschuß oder gegebenenfalls als Lösungsmittel verwendet.

Die Verbindungen I.2, in denen Hal für Iod steht, lassen sich außerdem in bekannter Weise [J. Chem. Soc. PTI, 416 (1976)] aus den Chloriden oder Bromiden durch Umsetzung mit Iodiden (z.B. Natriumiodid) in Aceton herstellen.

Das Benzylhalogenid dient als zentrales Zwischenprodukt zur Herstellung einer Vielzahl von Verbindungen I nach den folgenden Reaktionsschemata.
B.2 R⁴ = ggf. subst. Alkenyl

Durch Phoshorylierung der Benzylhalogenide I.2 und anschließende Wittig oder Wittig-Horner Umsetzung der Phosphorverbindungen I.5 mit Aldehyden gelangt man zu den entsprechenden Ethenylenderivaten I.6.

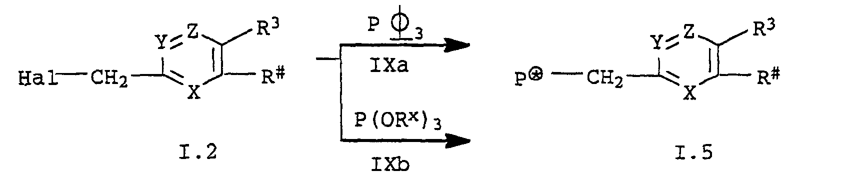

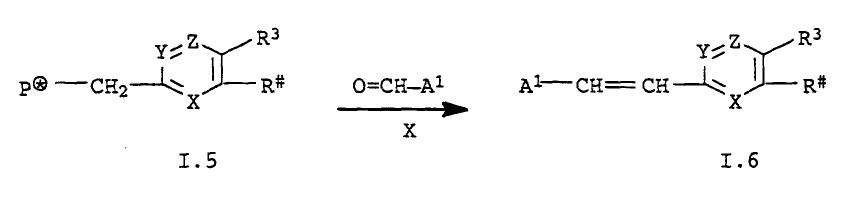

φ in der Formel IXa steht für Aryl, insbesondere Phenyl; R^{x} in der Formel IXb steht für Alkyl oder Aryl, insbesondere C₁-C₄-Alkyl oder Phenyl.

In den Formeln X und I.5 steht A¹ für ggf. subst. Alkyl, Alkenyl oder Alkinyl, oder ein ggf. subst. aromatisches Ringsystem.

Die Umsetzung der Halogenide I.2 mit den Phosphinen IXa bzw. den Phosphiten IXb erfolgt in an sich bekannter Weise [vgl. Houben-Weyl, 4. Aufl., Bd. XII/1, S. 79 f. und S. 433 f. (1963)].

Die Edukte I.2 und IXa bzw. IXb werden im allgemeinen in äquimolaren Mengen miteinander umgesetzt. Es kann für die Ausbeute vorteilhaft sein, IXa bzw. IXb in einem Überschuß bezogen auf I.2 einzusetzen.

Die so erhaltenen Phosphorderivate I.5 werden anschließend gemäß einer Wittig oder einer Wittig-Horner Reaktion bei Temperaturen von -30°C bis 60°C, vorzugsweise 0°C bis 40°C in einem inerten Lösungsmittel in Gegenwart einer Base mit einem Aldehyd der Formel X umgesetzt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Diethylester, Tetrahydrofuran, Dimethylsulfoxid und Dimethylformamid. Es können auch Gemische der genannten Lösungsmittel verwendet werden.

Als Basen kommen allgemein anorganische Verbindungen, z.B. Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriummethanolat, Kalium-t.-butylat, Natriumhydrid, Kaliumcarbonat und n-Butyllithium. Die Basen werden im allgemeinen in äquimolaren Mengen eingesetzt, sie können aber auch im Überschuß oder gegebenenfalls als Lösungsmittel verwendet werden.

Die Edukte I.5 und X werden im allgemeinen in äquimolaren Mengen miteinander umgesetzt. Es kann für die Ausbeute vorteilhaft sein, X in einem Überschuß bezogen auf I.5 einzusetzen.

In einer Abwandlung des vorstehenden Verfahrens erhält man die Verbindungen I.6 außerdem dadurch, daß man das Benzylhalogenid I.2 zunächst zum entsprechenden Benzaldehyd I.7 oxidiert und I.7 anschließend mit einer Phosphorverbindung Xa oder Xb im Sinne einer Wittig oder Wittig-Horner Reaktion umsetzt.

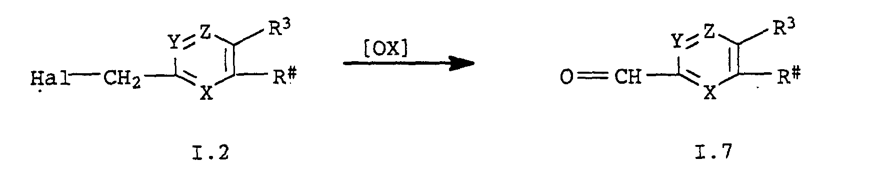

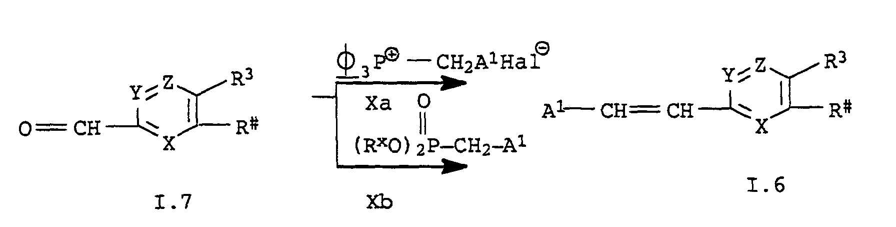

Als Oxidationsmittel [OX] dienen beispielsweise Methylmorpholin-N-oxidmonohydrat (vgl. EP-A 393 428) oder Dimethylsulfoxid [vgl. J. Chem. Soc. 1964, S. 520; J. Org. Chem. 24, 1792 (1959)].

Die anschließende Wittig bzw. Wittig-Horner Reaktion erfolgt nach den vorstehend beschriebenen Bedingungen.
B.3 R⁴ = über Oxy-, Mercapto- oder Amino- gebundener organischer Rest

Verbindungen I, in denen R⁴ für einen über eine Oxy-, Mercapto- oder Aminogruppe gebundenen organischen Rest steht, erhält man aus den entsprechenden Derivaten I.8 gemäß dem folgenden Reaktionsschema.

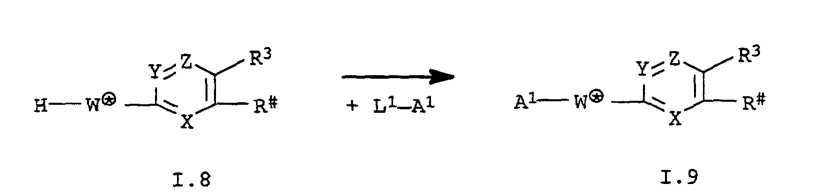

In den Formeln I.8 und I.9 bedeutet W^{*} Sauerstoff, Schwefel, Amino oder Alkylamino; A¹ hat die vorstehend gegebene Bedeutung.

L¹ in der Formel XI steht für eine nucleophil austauschbare Abgangsgruppe wie Halogen (z.B. Chlor, Brom und Iod) oder Alkyl- oder Arylsulfonat (z.B. Methylsulfonat, Trifluormethylsulfonat, Phenylsulfonat und 4-Methylphenylsulfonat).

Diese Umsetzung erfolgt üblicherweise in einem inerten Lösungsmittel in Gegenwart einer Base ggf. in Gegenwart eines Übergangsmetallkatalysators im Sinne einer Ullmann-Reaktion [vgl. Russ. Chem. Rev. 43, 679 (1974); J. Org. Chem. 29, 977 (1964)] bzw. im Sinne einer nucleophilen Substitutionsreaktion [vgl. J. Chem. Soc. 1942, 381, J. Heterocycl. Chem. 15, 1513 (1978)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Aceton und Dimethylformamid. Es können auch Gemische der genannten Lösungsmittel verwendet werden.

Die Edukte werden im allgemeinen in äquimolaren Mengen miteinander umgesetzt. Es kann für die Ausbeute vorteilhaft sein, L¹-A¹ in einem Über- oder Unterschuß bezogen auf I.8 einzusetzen.
B.4 R⁴ = -CR^{a}=N-W^{a}-A²

Verbindungen I, in denen R für eine Gruppe -CR^{a}=N-W^{a}-A² steht, erhält man beispielsweise aus den entsprechenden Ketonen I.10 durch Umsetzung mit einem O-substituierten Hydroxylamin oder -Hydroxylammonium Salz oder einem Hydrazin bzw. Hydrazoniumsalz gemäß den in EP-A 499 823 beschriebenen Methoden nach dem folgenden Reaktionsschema.

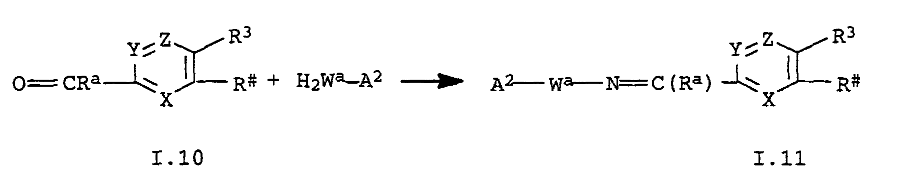

Die Reste R^{a}, W^{a} und A² in den Formeln I.10 und I.11 haben die folgende Bedeutung:
- R^{a}: Wasserstoff, Alkyl oder Halogenalkyl;
- W^{a}: Sauerstoff, Amino oder Alkylamino;
- A²: Wasserstoff, ggf. subst. Alkyl, Alkenyl oder Alkinyl.

Die Umsetzungsbedingungen entsprechen im allgemeinen und im besonderen den in der genannten Literatur beschriebenen Bedingungen.

Die Ketone I.10 (R^{a} ≠ H) erhält man aus den Aldehyden I.7 durch Oxidation zu den Carbonsäuren [vgl. Organikum, 15. Auflage, 447 (1977)], Umsetzung der Carbonsäuren zu den entsprechenden Carbonsäurehalogeniden [vgl. Organikum, 15. Auflage, 526 (1977)] und anschließende Umsetzung mit Zink-organischen Verbindungen [vg1. Org. React. (8), 28 (1954)].

Verbindungen I, in denen R⁴ für ggf. subst. Alkinyl steht, erhält man aus den Verbindungen I.15 (R=Halogen, insbesondere Brom und Jod) durch Umsetzung mit einem Acetylenderivat im Sinne einer Heck-Reaktion [vgl. J. Organomet. Chem. 93, 259 (1975)] im Gegenwart eines Übergangsmetallkatalysators [ÜM], z.B. einer Palladium- oder Nickelverbindung wie Diacetylpalladium, Palladiumdichlorid, Palladium-terta(triphenylphosphin) oder Nikkeldichlorid, in einem inerten Lösungsmittel (z.B. Dimethylformamid, Acetonitril, Tetrahydrofuran oder Toluol) und in Gegenwart einer Base (z.B. Kaliumcarbonat, Natriumhydrid, Diethylamin, Triethylamin oder Pyridin).

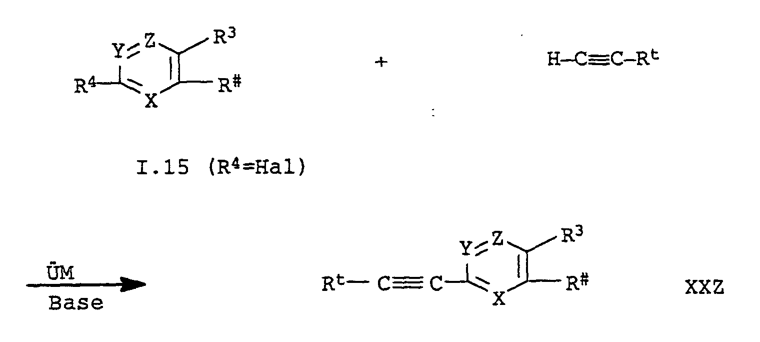

R^{t} bedeutet hierbei Wasserstoff, Alkyl oder einen ggf. subst. aromatischen Ring.

Besonders bevorzugt sind Phenylessigsäurealkylester der Formel I, in der R⁴ für eine Gruppe A¹-Y¹- steht, wobei A¹ und Y¹ die folgende Bedeutung haben:
- Y¹: eine direkte Bindung, Sauerstoff, Schwefel, Amino oder Alkylamino;
- A¹: C₁-C₁₀-Alkyl, C₂-C₁₀-Alkenyl oder C₂-C₁₀-Alkinyl,
wobei diese Gruppen partiell oder vollständig halogeniert sein können und einen der folgenden Reste tragen können:
unsubstituiertes Phenyl oder durch Halogen, Cyano, Nitro, C₁-C₄-Alkyl, C₁-C₄-Halogenalkyl, C₁-C₄-Alkoxy,
C₁-C₄-Halogenalkoxy, C₁-C₄-Alkylamino, Di-C₁-C₄-Alkylamino und C₁-C₄-Alkylthio substituiertes Phenyl,
ein- bis dreikerniges aromatisches Ringsystem enthaltend 6-14 Kohlenstoffringglieder, 5-gliedriges Hetaryl enthaltend ein bis vier Stickstoffatome oder ein bis drei Stickstoffatome und ein Schwefel oder Sauerstoffatom, oder 6-gliedriges Hetaryl enthaltend ein bis drei Stickstoffatome,
wobei die (hetero)aromatischen Gruppen durch Halogen, Cyano, Nitro, C₁-C₄-Alkyl, C₁-C₄-Halogenalkyl,
C₁-C₄-Alkoxy, C₁-C₄-Halogenalkoxy, C₁-C₄-Alkylamino,
Di-C₁-C₄-Alkylamino und C₁-C₄-Alkylthio substituiert sein und/oder einen oder zwei der folgenden Reste tragen können: Formyl oder CRⁱⁱⁱ=NOR^{iv},
wobei Rⁱⁱⁱ Wasserstoff oder C₁-C₄-Alkyl und R^{iv} C₁-C₄-Alkyl und Phenyl-C₁-C₄-alkyl bedeuten,
oder Phenyl, Pyridyl, Pyrimidyl oder Triazinyl, wobei diese Gruppen durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio substituiert sein können;

Gleichermaßen werden Phenylessigsäurealkylester der Formel I bevorzugt, in der R⁴ für eine Gruppe A²-W^{a}N=CR^{a}- steht, wobei A², W^{a} und R^{a} die folgende Bedeutung haben:
- R^{a}: Wasserstoff, C₁-C₁₀-Alkyl oder C₁-C₁₀-Halogenalkyl;
- W^{a}: Sauerstoff, Amino oder C₁-C₁₀-Alkylamino;
- A²: Wasserstoff,
C₁-C₁₀-Alkyl, C₂-C₁₀-Alkenyl oder C₂-C₁₀-Alkinyl,
wobei diese Gruppen partiell oder vollständig halogeniert sein können und einen der folgenden Reste tragen können: unsubstituiertes Phenyl oder durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino oder C₁-C₄-Alkylthio substituiertes Phenyl.

Bei den Verbindungen der Formel I hat die Kombination der Gruppen X, Y und Z die folgende Bedeutung:
X, Y und Z bedeuten =CR³-; oder
X bedeutet =N- und Y und Z bedeuten =CR³-; oder
Y bedeutet =N- und X und Z bedeuten =CR³-; oder
Z bedeutet =N- und X und Y bedeuten =CR³-; oder
X bedeutet =CR³- und Y und Z bedeuten =N-; oder
Y bedeutet =CR³- und X und Z bedeuten =N-; oder
Z bedeutet =CR³- und X und Y bedeuten =N-; oder
X, Y und Z bedeuten =N-.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
   Halogenalkyl: geradkettige oder verzweigte Alkylgruppen mit 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 10 Kohlenstoffatomen (wie vorstehend genannt), welche über ein Sauerstoffatom (-O-) an das Gerüst gebunden sind;
Halogenalkoxy: geradkettige oder verzweigte Halogenalkylgruppen mit 1 bis 10 Kohlenstoffatomen (wie vorstehend genannt), welche über ein Sauerstoffatom (-O-) an das Gerüst gebunden sind; Alkylthio: geradkettige oder verzweigte Alkylgruppen mit 1 bis 10 oder 1 bis 4 Kohlenstoffatomen (wie vorstehend genannt), welche über ein Schwefelatom (-S-) an das Gerüst gebunden sind;
Alkylamino: eine geradkettige oder verzweigte Alkylgruppe mit 1 bis 10 Kohlenstoffatomen (wie vorstehend genannt), welche über eine Aminogruppe (-NH-) an das Gerüst gebunden ist;
Alkenyl: ungesättigte, geradkettige oder verzweigte Kohlenwasserstoffreste mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-lpropenyl und 1-Ethyl-2-methyl-2-propenyl;
Alkinyl: geradkettige oder verzweigte Kohlenwasserstoffgruppen mit 2 bis 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-i butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Aryl: ein ein- bis dreikerniges aromatisches Ringsystem enthaltend 6 bis 14 Kohlenstoffringglieder, z.B. Phenyl, Naphthyl und Anthracenyl;
aromatisches Ringsystem, welches neben Kohlenstoffringgliedern Heteroatome aus der gruppe Sauerstoff, Schwefel und Stickstoff enthalten kann: Aryl wie vorstehend genannt oder ein- oder zweikerniges Heteroaryl, z.B.
   - 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol -3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;

Der Zusatz "*ggf*. *subsk.*" in Bezug auf *Alkyl-, Alkenyl- und Alkinylgruppen* soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor oder Brom) ersetzt sein können und/ oder einen bis drei (vorzugsweise einen) der folgenden Reste tragen können:
unsubstituiertes oder durch übliche Gruppen substituiertes Phenyl.

Der Zusatz "*ggf. subst*" in Bezug auf die *cyclischen (gesättigten, ungesättigten oder aromatischen) Gruppen* soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor oder Brom, insbesondere Fluor oder Chlor) ersetzt sein können und/oder einen bis vier (insbesondere einen bis drei) der folgenden Reste
Cyano, Nitro, Hydroxy, Amino, Carboxyl, Aminocarbonyl, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, Alkylthio, Halogenalkylthio, Alkylamino, Dialkylamino, Alkylcarbönyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;
unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten
und oder einen oder zwei (insbesondere einen) der folgenden Reste
Formyl oder CRⁱⁱⁱ=NOR^{iv}, wobei Rⁱⁱⁱ Wasserstoff oder Alkyl und R^{iv} Alkyl und Arylalkyl bedeutet und wobei die genannten Alkylgruppen vorzugsweise 1 bis 6 Kohlenstoffatome, insbesondere 1 bis 4 Kohlenstoffatome, enthalten und Aryl insbesondere Phenyl bedeutet, welches unsubstituiert ist oder durch übliche Gruppen substituiert sein kann, tragen kann.

Unter üblichen Gruppen sind insbesondere die folgenden Substituenten zu verstehen: Halogen, Cyano, Nitro, C₁-C₄-Alkyl, C₁-C₄-Halogenalkyl, C₁-C₄-Alkyoxy, C₁-C₄-Halogenalkoxy, C₁-C₄-Alkylamino, Di-C₁-C₄-Alkylamino und C₁-C₄-Alkylthio.

Gleichermaßen werden Verbindungen der Formel I.B bevorzugt, 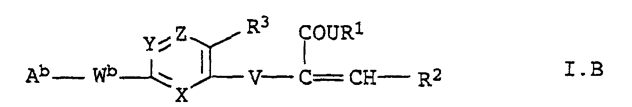 in denen die Substituenten die folgende Bedeutung haben:
- W^{b}: eine direkte Bindung, Sauerstoff, Schwefel, Amino oder Alkylamino,
- A^{b}: Aryl oder Heteroaryl, welches durch übliche Gruppen substituiert sein kann und/oder einen oder zwei (insbesondere einen) der folgenden Reste tragen kann: Formyl, CRⁱⁱⁱ=NOR^{iv} oder unsubstituiertes oder durch übliche Gruppen substituiertes Aryl (insbesondere Phenyl) oder Heteroaryl (insbesondere Pyridyl, Pyrimidyl oder Triazinyl).

Insbesondere werden Verbindungen I.B bevorzugt, in denen W^{b} für Sauerstoff steht.

Daneben werden Verbindungen I.B bevorzugt, in denen W^{b} für eine direkte Bindung steht.

Außerdem werden Verbindungen I.B besonders bevorzugt, in denen W^{b} für Schwefel steht.

Insbesondere werden auch Verbindungen I.B bevorzugt, in denen W^{b} für ggf. subst. Aryl steht.

Außerdem werden Verbindungen I.B besonders bevorzugt, in denen W^{b} für einen ggf. subst. 6-gliedrigen Heteroarylring steht.

Gleichermaßen besonders bevorzugt sind Verbindungen I.B, in denen A^{b} für einen ggf. subst. Aryl- oder Hetarylring aus der Gruppe Phenyl, Pyridyl, Pyrimidyl, Pyrazinyl und 1,3,5-Triazinyl steht.

Daneben werden Verbindungen I.B besonders bevorzugt, in denen A^{b} für ggf. subst. 4-Pyridinyl steht, welches in 6-Position einen ggf. subst. Phenoxyrest trägt.

Des weiteren werden Verbindungen I.B besonders bevorzugt, in denen A^{b} für ggf. subst. Pyrimidyl steht, welches einen ggf. subst. Pyridinyloxyrest trägt.

Außerdem werden Verbindungen I.B besonders bevorzugt, in denen A^{b} für ggf. subst. 1,3,5-Triazinyl steht, welches in 4-Position einen ggf. subst. Phenoxyrest trägt.

Außerdem werden Verbindungen der Formel I.E bevorzugt, 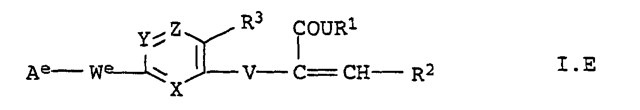 in denen die Substituenten die folgende Bedeutung haben:
- W^{e}: CR^{a}=N-T^{a}-#,
- R^{a}: Wasserstoff, C₁-C₁₀-Alkyl oder C₁-C₁₀-Halogenalkyl,
- T^{a}: Sauerstoff, Amino oder C₁-C₁₀-Alkylamino,
- -#: Bindung zu A^{e},
- A^{e}: Wasserstoff,
C₁-C₁₀-Alkyl C₂-C₁₀-Alkenyl oder C₂-C₁₀-Alkinyl,
wobei diese Gruppen partiell oder vollständig halogeniert sein können und einen der folgenden Reste tragen können: unsubstituiertes Phenyl oder durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino oder C₁-C₄-Alkylthio substituiertes Phenyl.

Insbesondere werden Verbindungen I.E bevorzugt, in denen W^{e} für CH(CH₃)=N-O-# steht.

Außerdem werden Verbindungen I.E besonders bevorzugt, in denen W^{e} für CH(CH₃)=N-NH-# steht.

Gleichermaßen besonders bevorzugt sind Verbindungen I.E, in denen W^{e} für CH=N-N(CH₃)-# steht.

Insbesondere werden auch Verbindungen I.E bevorzugt, in denen A^{e} für Alkyl steht.

Außerdem werden Verbindungen I.E besonders bevorzugt, in denen A^{e} für ggf. subst. Arylalkyl steht.

Im Hinblick auf ihre biologische Wirksamkeit können sowohl die Isomerengemische als auch die reinen Isomere eingesetzt werden, wobei die reinen Isomere eine bessere Wirkung entfalten können als die Isomerengemische.

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungsformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfsi lösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-form-aldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), AmmoniakKomplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol,2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylaminobenzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis- (3-ethoxycarbonyl-2-thioureido)-benzol, 1,2-Bis-(3-methoxycarbonyl-2-thioureido)-benzol, sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

### Beispiel 1

### α-(3-Jodphenoxy)-crotonsäuremethylester

Zu einer Lösung von 3 g (0,055 Mol) KOH in 30 ml Dimethylformamid wird bei 0 bis 5°C eine Lösung aus 11 g (0,05 mol) 3-Jodphenol zugetropft. Bei dieser Temperatur werden 9 g (0,05 mol) 3-Bromcrotonsäuremethylester hinzugegeben und die Lösung für eine Stunde bei Raumtemperatur nachgerührt. Man versetzt die Mischung mit Eiswasser und extrahiert mit Methyl-tert.Butylether, trocknet über Na₂SO₄ und engt im Vakuum ein. Der Rückstand wird durch Chromatographie an Kieselgel (Cyclohexan/Essigsäureethylester: 99/1) gereinigt. Man erhält 9,7 g (61 % der Theorie) der Titelverbindung als Öl.
¹HNMR (COCL₃, δ-Skala) = 1,83 (d, 3H); 3,73 (s, 3H); 6,75 (q, 1H) ; 6,80-7,40 (m, 4H).

### Beispiel 2

### 2-(4'Chloro-biphenyl-4'-yloxy)-but-2-en-säuremethylester

Eine Mischung aus 2,2 g (0,07 mol)-α-(3-Jodphenoxy)-crotonsäuremethylester, 1,8 g (0,021 mol) NaHCO₃, 1,1 g (0,007 mol) 4-Chlorphenylboronsäure und 20 mg Palladium-tetrahis-triphenylphosphin in 30 ml Wasser und 30 ml Dimethoxyethan wird für 3 Stunden unter Rückfluß erhitzt. Die Aufarbeitung erfolgt durch Extraktion mit Diethylether, Trocknung über Na₂SO₄ und Eindampfen im Vakuum.

Der Rückstand wird durch Chromatographie an Kieselgel (Cyclohexan/Essigsäurethylester: 98/2) gereinigt. Man erhält 2,2 g (90 % der Theorie) der Titelverbindung als Öl.
¹H-NMR (COCL₃, δ-Skala) = 1,82 (d, 3H); 3,8 (s, 3H); 6,73 (q, 1H) ; 6,82-7,55 (m, 8H).

### Beispiel 3

### 2-[(3'-Acetyl-phenyl)-methyl-amino]-but-2-en-säuremethylester

Eine Mischung aus 4,7 g (0,032 mol 1-(3-Methylamine-phenyl)-ethanon, 100 mg p-Toluolsulfonsäure und 3,7 g (0,07 mol) 2-Oxo-buttersäuremethylester werden in 100 ml Toluol für 4 Stunden am Wasserabscheider erhitzt. Die im Vakuum eingedampfte Reaktionsmischung wird durch Säulenchromatographie über Kieselgel (Cyclohexan/Essigsäureethylester: 95/5) gereinigt. Man erhält 3,4 g (69 % der Theorie) der Titelverbindung als Feststoff (Fp = 73°-76°C).
¹H-NMR: (COCL₃, δ-Skala) = 1,81 (d, 3H), 2,61 (s, 3H); 3,09 (s, 3H); 3,79 (s, 3H); 6,76 (g, 1H); 7,10-7,60 (m, 4H).

### Beispiel 4

### 2-{3-[1-(1-m-Tolylethoxyimino)-ethyl]-phenyl-N-methyl-amino-butensäuremethylester

Eine Mischung aus 1,2 g 2-[(3'-Acetyl-phenyl)-methylamino]-but-2-en-säuremethylester, 0,72 g (0,005 mol) 1-(4-Methylphenyl)-ethylhydroxylamin, 2 g Mg SO₄ und 100 mg Dowex® in 100 ml trockenem Methanol wird für 3 Stunden unter Rückfluß erhitzt.

Die Aufarbeitung erfolgt durch Eindampfen der Reaktionslösung im Vakuum und anschließende Chromatographie an Kieselgel (Cyclohexan/Essigsäureethylester: 97/3). Man erhält 1,2 g (63 % der Theorie) der Titelverbindung als Öl.
¹HNMR (COCL₃, δ-Skala) = 1,62 (d, 3H); 1,82 (d, 3H); 2,21 (s, 3H); 2,29 (s, 3H); 3,05 (s, 3H); 3,75 (s, 3H); 5,24 (g, 1H); 6,60-7,25 (m, 8H).

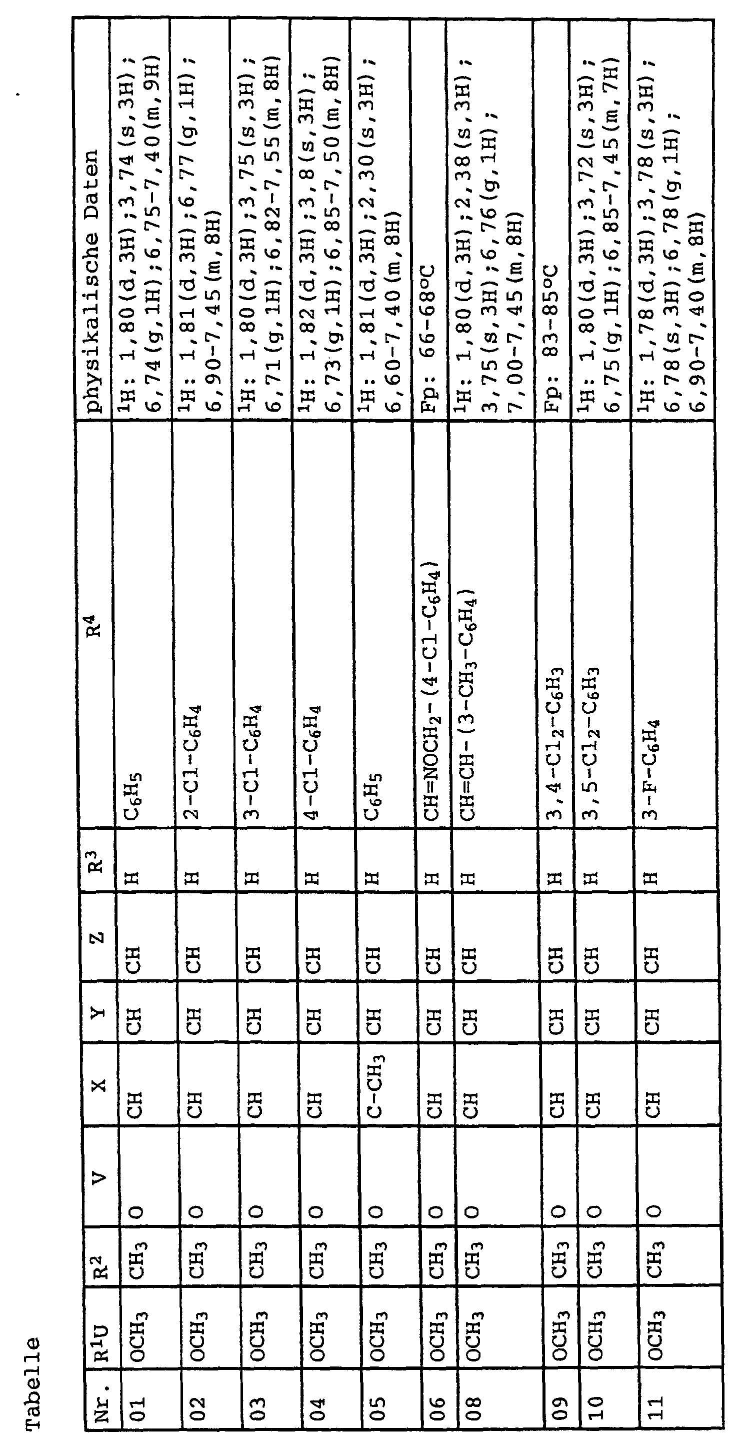

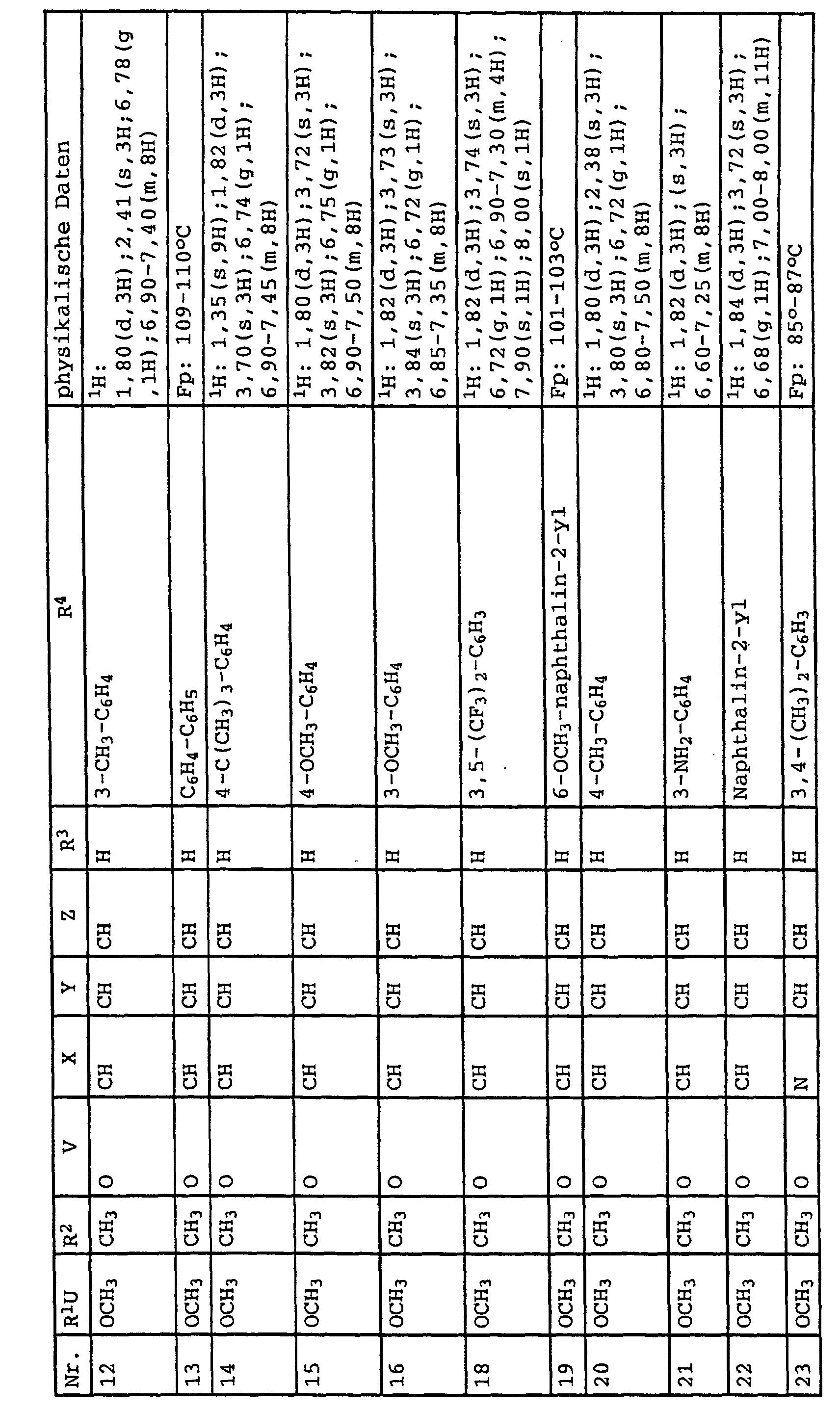

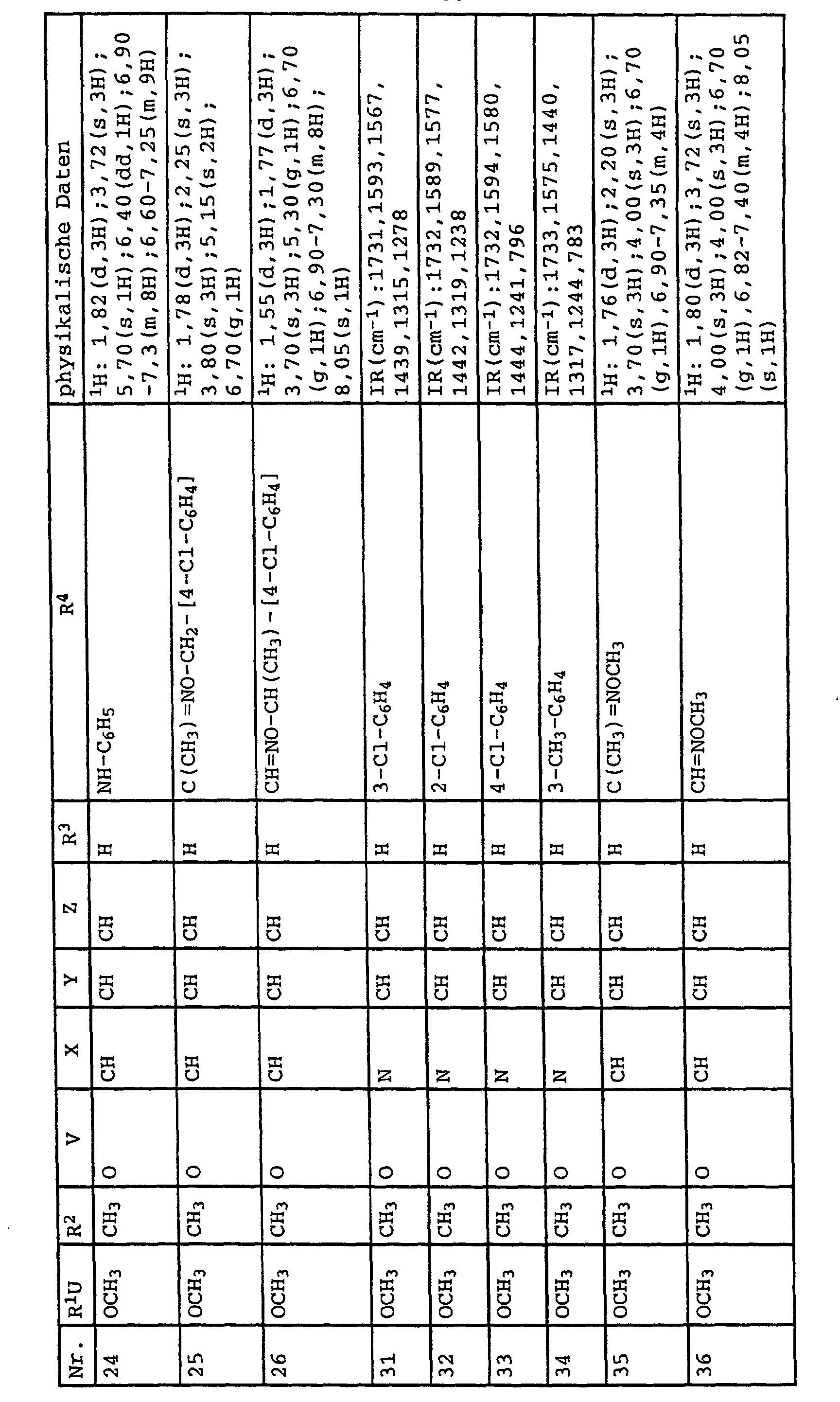

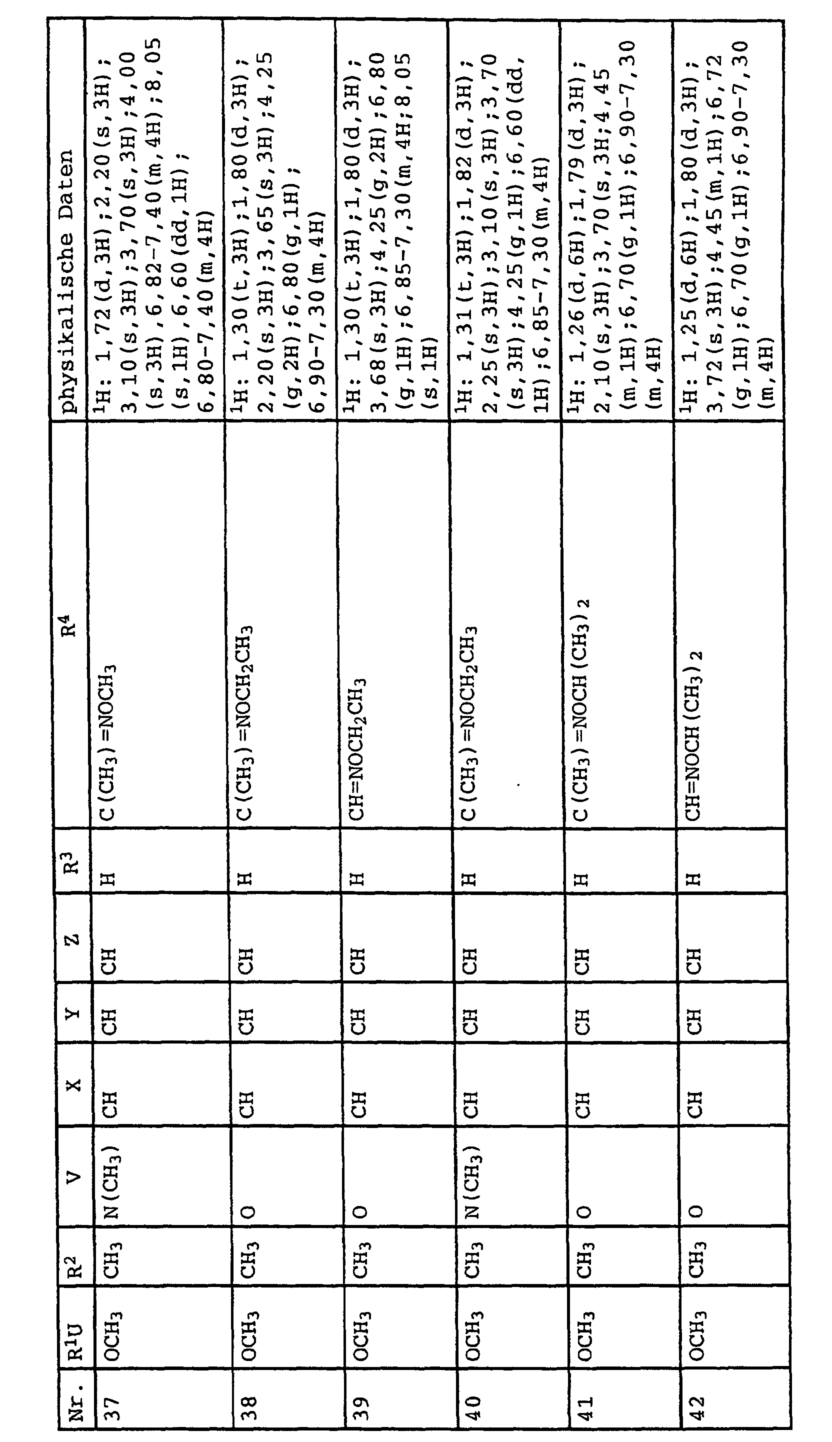

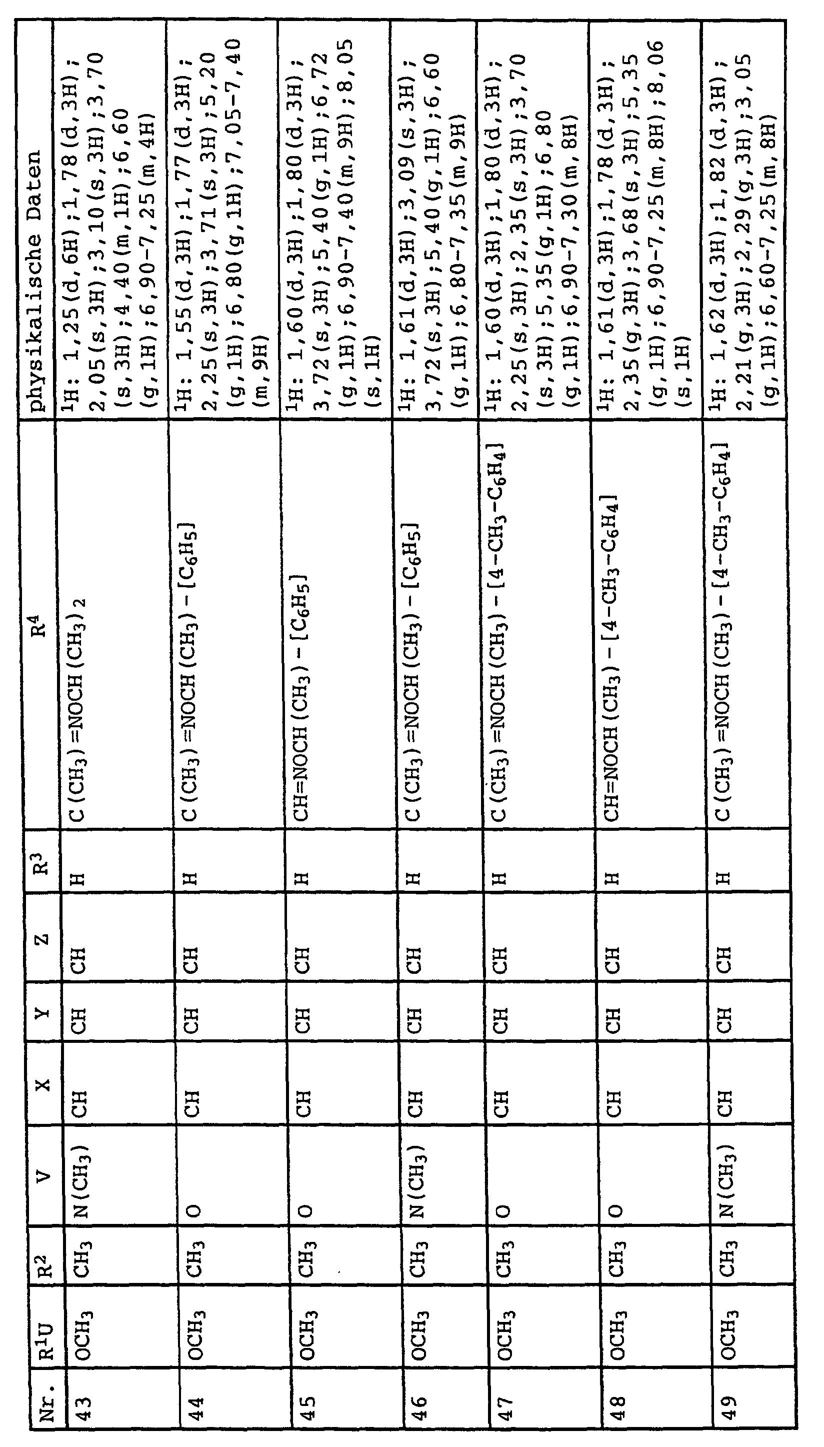

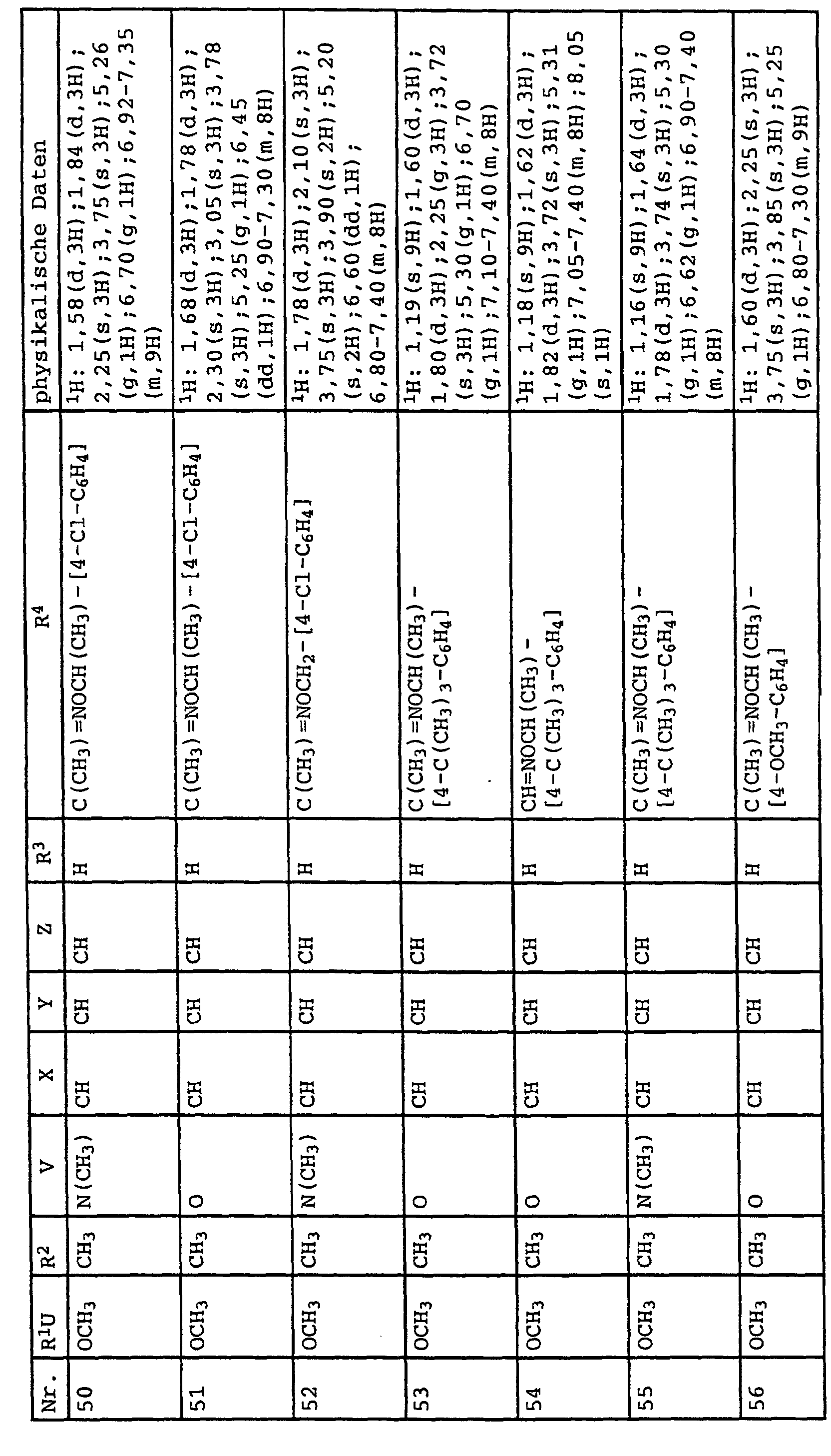

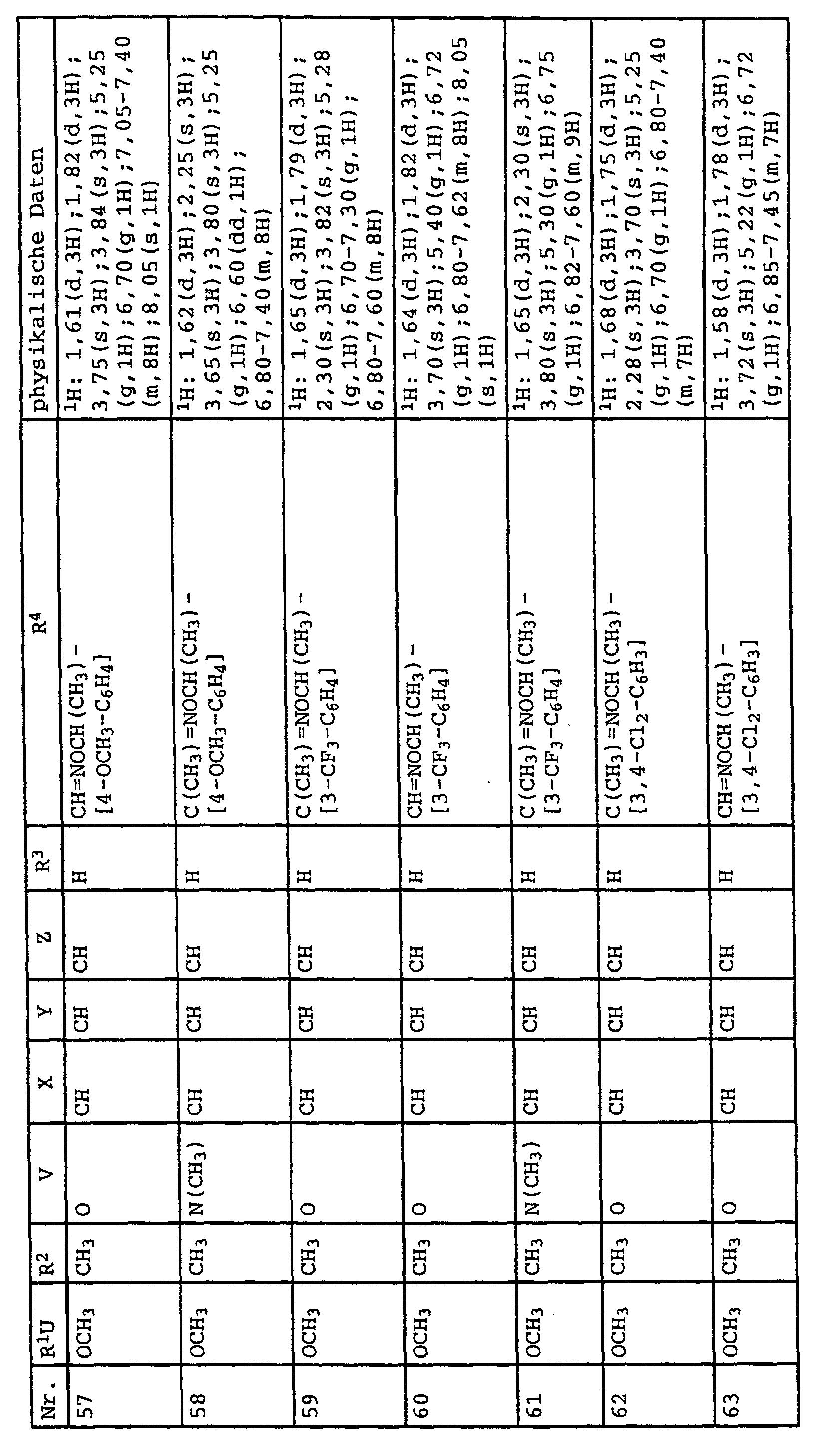

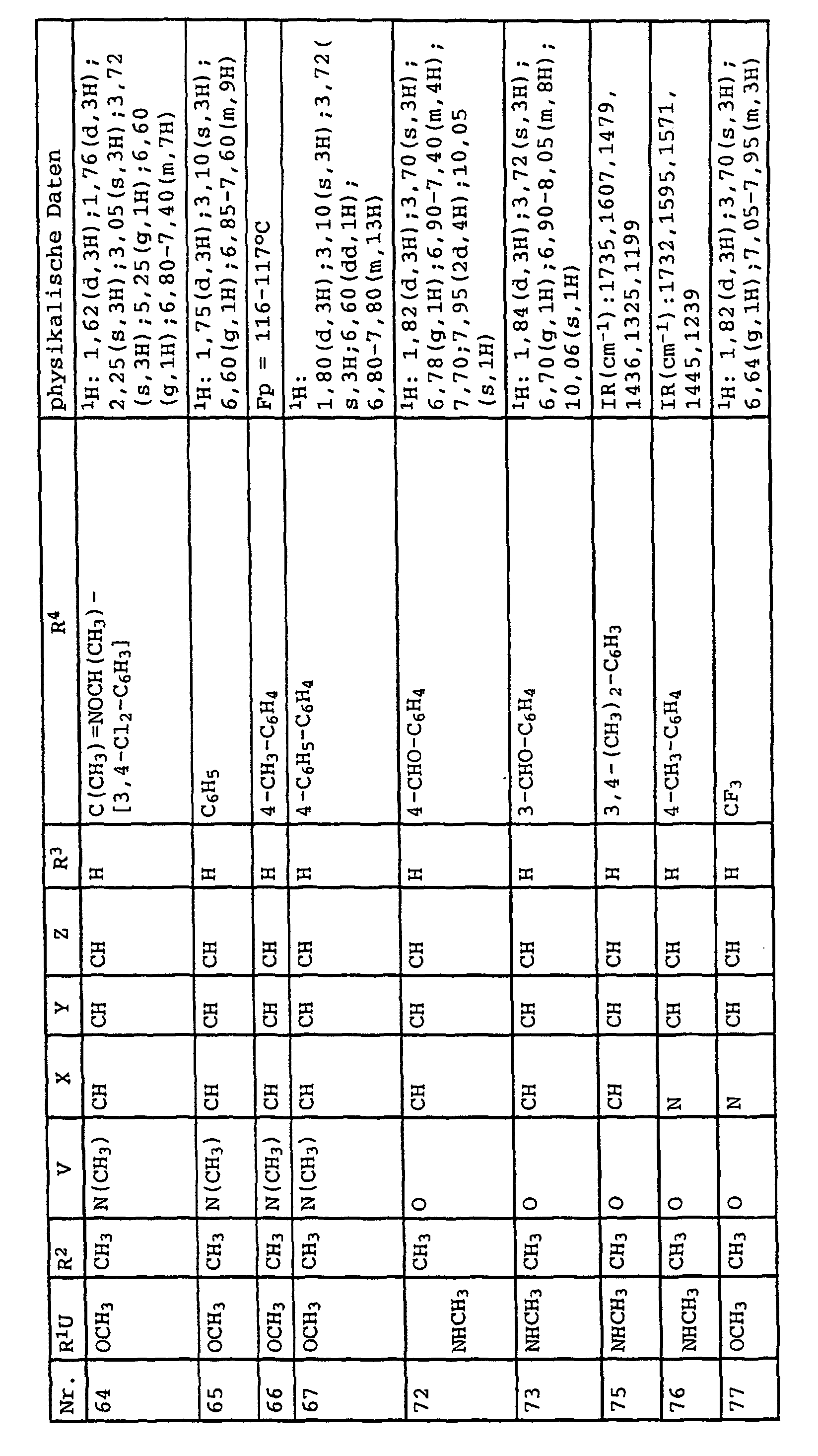

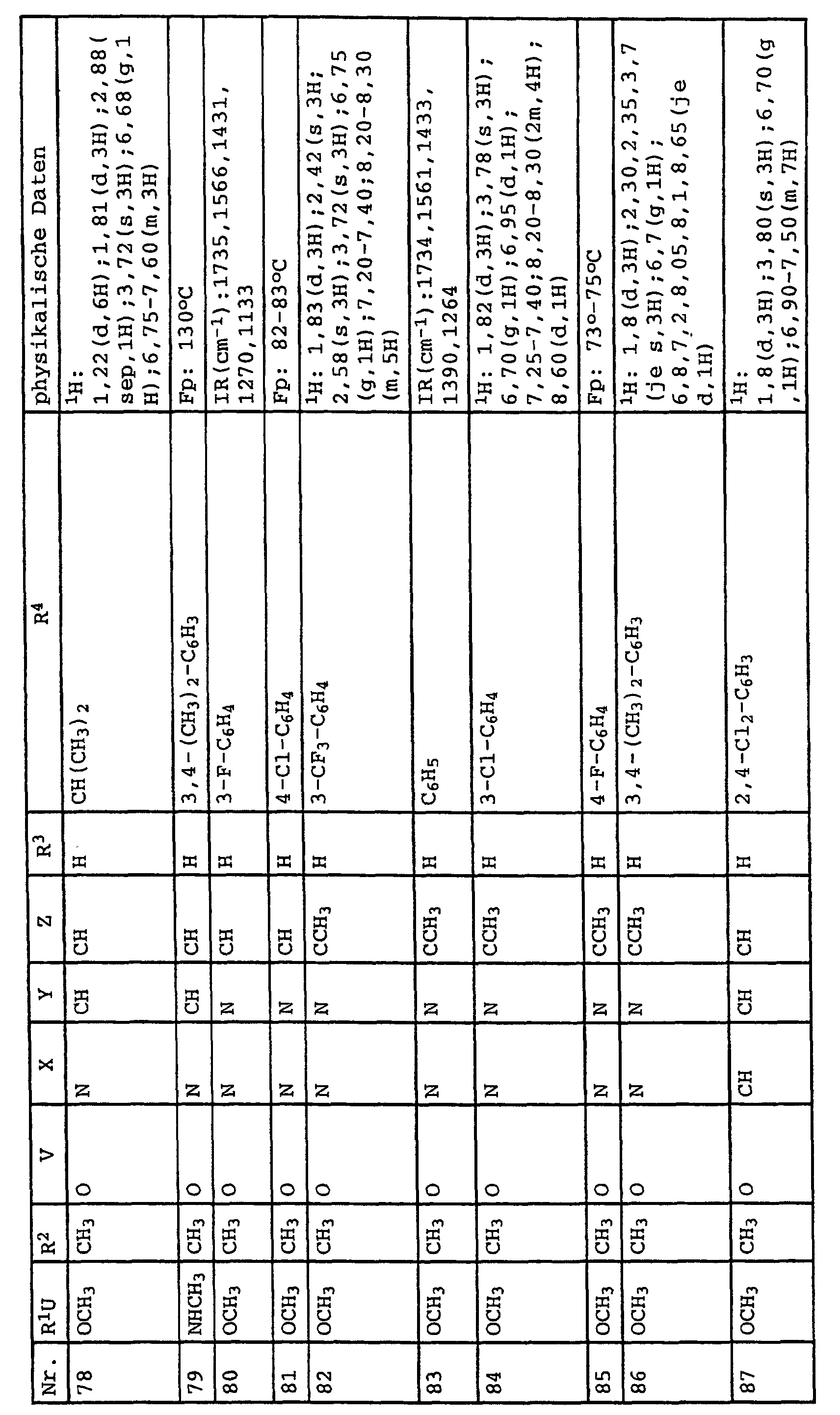

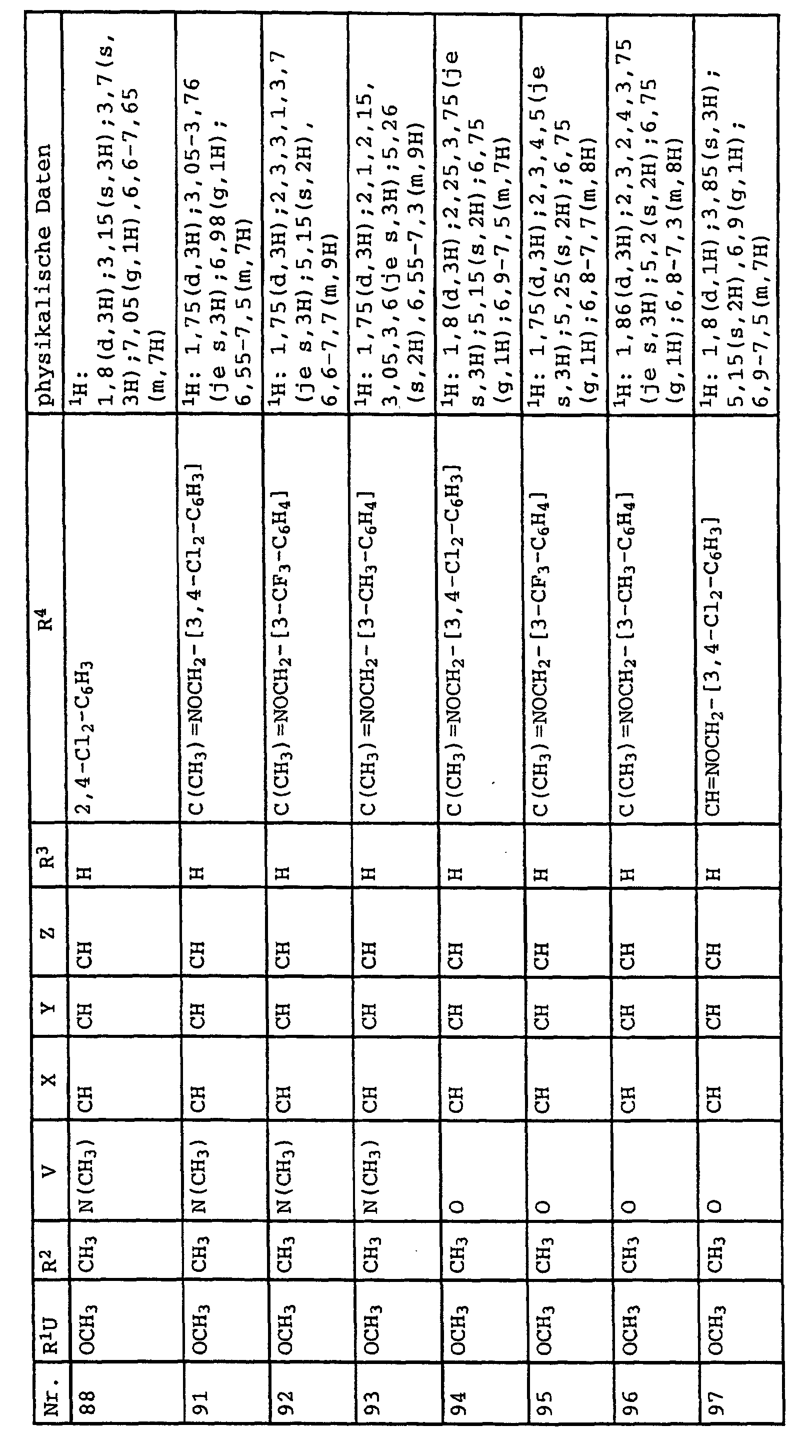

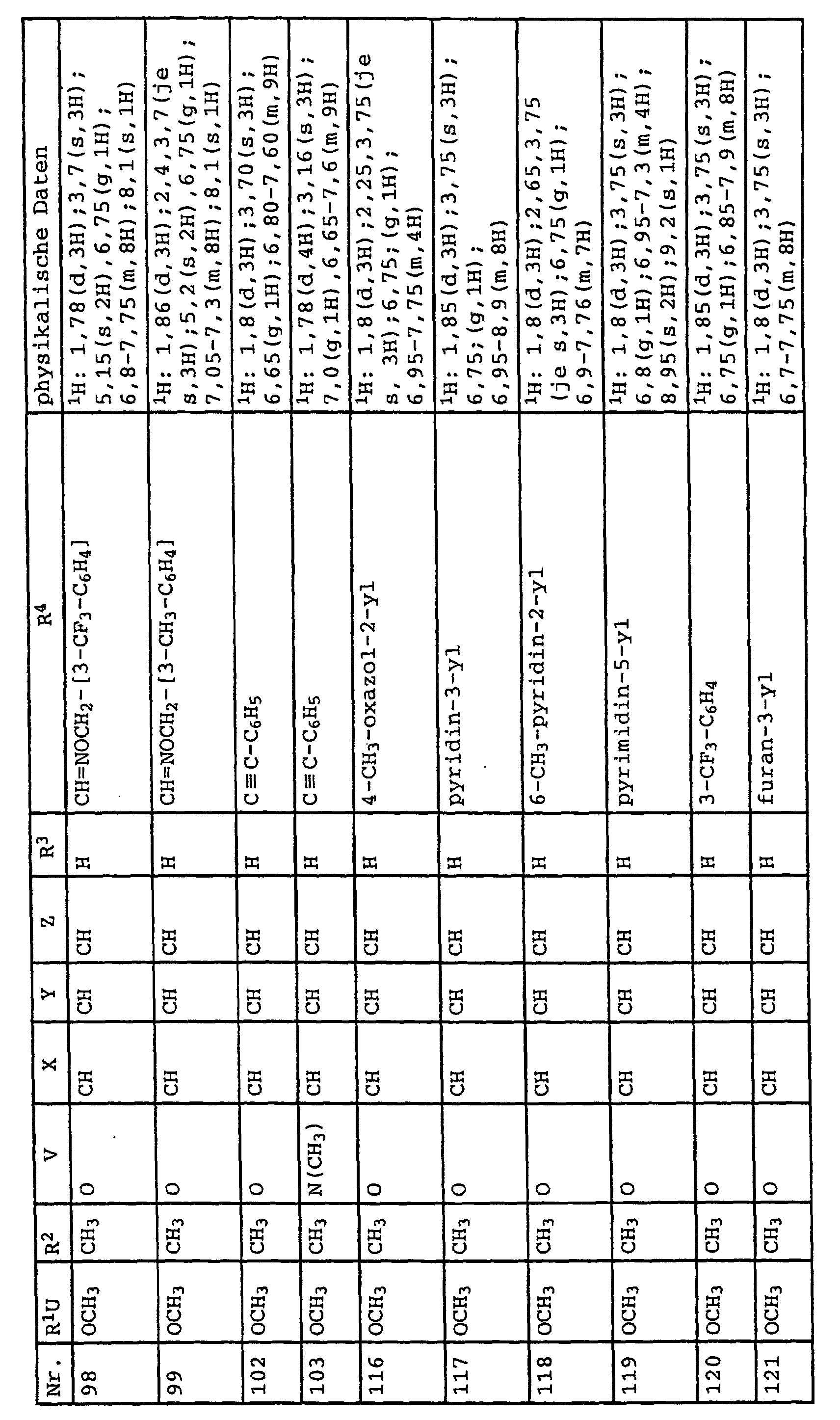

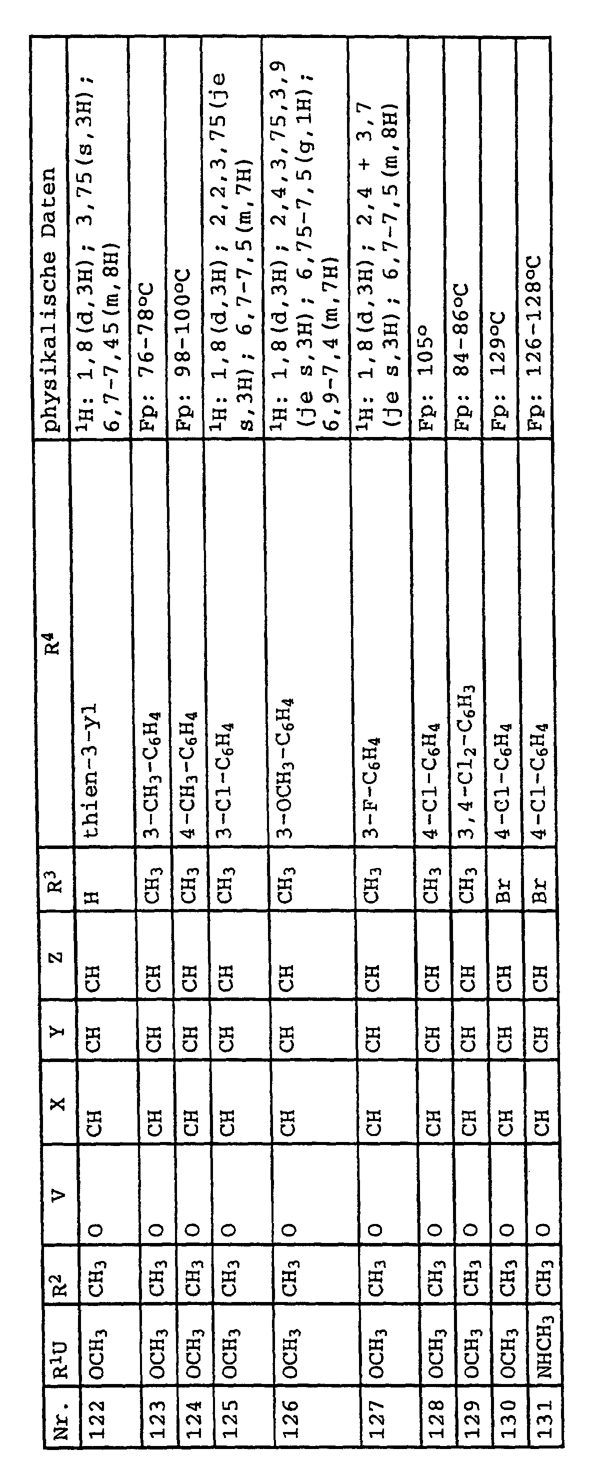

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 20%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Als Vergleichswirkstoffe dienten die Verbindungen **A** (Beispiel 1 aus EP-A 383 117) und **B** (Beispiel 50 aus EP-A 471 261).

### Wirkung gegen Erysiphe graminis var. tritici (Weizenmehltau)

Blätter von Weizenkeimlingen (Sorte "Frühgold") wurden zunächst mit der wäßrigen Aufbereitung der Wirkstoffe (Aufwandmenge: 16 ppm) behandelt. Nach ca. 24 h wurden die Pflanzen mit Sporen des Weizenmehltaus (*Erysiphe graminis var. tritici*) bestäubt. Die so behandelten Pflanzen wurden anschließend für 7 Tage bei 20-22$C und einer relativen Luftfeuchtigkeit von 75-80% inkubiert. Anschließend wurde das Ausmaß der Pilzentwicklung ermittlelt.

In diesem Test zeigten die mit den erfindungsgemäßen Verbindungen 4, 9, 13, 23, 25, 33, 59, 66, 86, 89, 95, 96, 118 und 123 bis 130 behandelten Pflanzen einen Befall von 15% und weniger während die mit dem Vergleichswirkstoff **A** behandelten Pflanzen zu 50% befallen waren. Die mit dem Vergleichswirkstoff **B** behandelten Pflanzen zeigten ebenso wie die unbehandelten (Kontroll-) Pflanzen einen Befall von 70%.

### Wirkung gegen Pyricularia oryzae (Reisbrand)

Reiskeimlinge (Sorte: "Tai Nong 67") wurden mit der Wirkstoffaufbereitung tropfnaß gespritzt. Nach 24 Stunden wurden die Pflanzen mit einer wäßrigen Sporensuspension des Pilzes *Pyricularia oryzae* besprüht und 6 Tage bei 22-24 $C bei einer relativen Luftfeuchtigkeit von 95-99 % bewahrt. Die Beurteilung erfolgte visuell.

In diesem Test zeigten die mit den erfindungsgemäßen Verbindungen 9, 23, 33, 40, 43, 44, 46, 47, 49, 51, 52, 56, 58, 59, 60 bis 62, 64 bis 67, 75, 91 bis 96, 118 und 123 bis 129 behandelten Pflanzen einen Befall von 25% und weniger während die mit den Vergleichswirkstoffen **A** und **B** behandelten Pflanzen ebenso wie die unbehandelten (Kontroll-) Pflanzen einen Befall von 80% zeigten.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. (Het)aryloxy-, -thio- und -aminocrotonate der Formel I 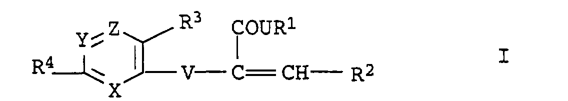 in der die Substituenten die folgenden Bedeutung haben:
U Sauerstoff (-O-), Schwefel (-S-) oder Amino (-NH-);
V Sauerstoff (-O-), Schwefel (-S-), Amino (-NH-) oder C₁-C₁₀-Alkylamino [-N(C₁-C₁₀-Alkyl)-];
X,Y,Z unabhängig voneinander =N- oder =CR³-;
R¹, R² unabhängig voneinander C₁-C₄-Alkyl;
R³ Wasserstoff, Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
R⁴ eine Gruppe A¹-Y¹ oder eine Gruppe A²-W^{a}-N=CR^{a}-;
Y¹ eine direkte Bindung, Sauerstoff, Schwefel, Amino oder C₁-C₁₀-Alkylamino;
A¹ C₁-C₁₀-Alkyl, C₂-C₁₀-Alkenyl oder C₂-C₁₀-Alkinyl,
wobei diese Gruppen partiell oder vollständig halogeniert sein können und einen der folgenden Reste tragen können: unsubstituiertes Phenyl oder durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio substituiertes Phenyl,
ein- bis dreikerniges aromatisches Ringsystem enthaltend 6-14 Kohlenstoffringglieder, 5-gliedriges Hetaryl enthaltend ein bis vier Stickstoffatome oder ein bis drei Stickstoffatome und ein Schwefel oder Sauerstoffatom, oder 6-gliedriges Hetaryl enthaltend ein bis drei Stickstoffatome,
wobei die (hetero)aromatischen Gruppen durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio substituiert sein und/oder einen oder zwei der folgenden Reste tragen können: Formyl oder CRⁱⁱⁱ=NOR^{iv},
wobei Rⁱⁱⁱ Wasserstoff oder C₁-C₄-Alkyl und R^{iv} C₁-C₄-Alkyl und Phenyl-C₁-C₄-alkyl bedeuten, oder Phenyl, Pyridyl, Pyrimidyl oder Triazinyl,
wobei diese Gruppen durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio substituiert sein können;
R^{a} Wasserstoff, C₁-C₁₀-Alkyl oder C₁-C₁₀-Halogenalkyl;
W^{a} Sauerstoff, Amino oder C₁-C₁₀-Alkylamino;
A² Wasserstoff,
C₁-C₁₀-Alkyl, C₂-C₁₀-Alkenyl oder C₂-C₁₀-Alkinyl,
wobei diese Gruppen partiell oder vollständig halogeniert sein können und einen der folgenden Reste tragen können: unsubstituiertes Phenyl oder durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino oder C₁-C₄-Alkylthio substituiertes Phenyl.

2. Verbindungen der Formel I gemäß Anspruch 1, in denen U Sauerstoff und V Sauerstoff oder Amino bedeutet.

3. Verbindungen der Formel I gemäß Anspruch 1, in denen R¹ für Methyl steht.

4. Verbindungen der Formel I gemäß Anspruch 1, in denen R² für Methyl steht.

5. Verfahren zur Herstellung der Verbindungen I gemäß Anspruch 1, in denen V Sauerstoff oder Schwefel bedeutet, dadurch gekennzeichnet, daß man einen entsprechenden Alkohol bzw. ein entsprechendes Thiol der Formel IIa 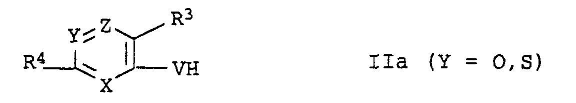 in an sich bekannter Weise in Gegenwart einer Base mit einem Crotonat der Formel III 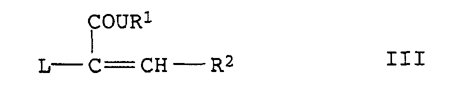 in der L für eine nucleophil austauschbare Abgangsgruppe steht, umsetzt.

6. Verfahren zur Herstellung der Verbindungen I gemäß Anspruch 1, in denen V für Amino oder C₁-C₁₀-Alkylamino steht, dadurch gekennzeichnet, daß man ein entsprechendes Amin der Formel IIb 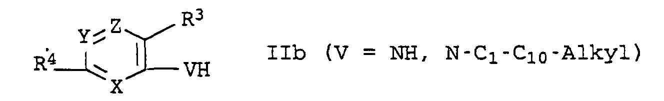 in an sich bekannter Weise in Gegenwart von Protonensäuren mit einer α-Ketocarbonylverbindung der Formel IV 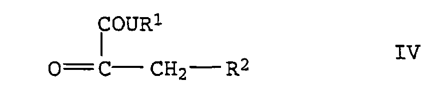 umsetzt.

7. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

8. Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

9. Verfahren zur Bekämpfung von tierischen Schädlingen, dadurch gekennzeichnet, daß man die Schädlinge oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

1. A (Het)aryloxy-, -thio- or -aminocrotonate of the formula I 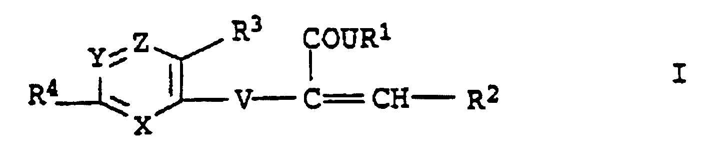 in which the substituents have the following meanings:
U is oxygen(-O-), sulfur, (-S-) or amino (-NH-);
V is oxygen (-O-), sulfur, (-S-), amino (-NH-) or C₁-C₁₀-alkylamino [-N(C₁-C₁₀-alkyl)-];
X, Y, Z independently of one another are =N- or =CR³-;
R¹, R² independently of one another are C₁-C₄-alkyl;
R³ is hydrogen, cyano, nitro, halogen, C₁-C₄-alkyl, C₁-C₄-haloalkyl, C₁-C₄-alkoxy, C₁-C₄-haloalkoxy, C₁-C₄-alkylthio and C₁-C₄-haloalkylthio;
R⁴ is a group A¹-Y¹ or a group A²-W^{a}-N=CR^{a}-;
Y¹ is a direct bond, oxygen, sulfur, amino or C₁-C₁₀-alkylamino;
A¹ is C₁-C₁₀-alkyl, C₂-C₁₀-alkenyl or C₂-C₁₀-alkynyl,
it being possible for these groups to be partially or fully halogenated and to have attached to them one of the following radicals: unsubstituted phenyl or phenyl which is substituted by halogen, cyano, nitro, C₁-C₄-alkyl, C₁-C₄-haloalkyl, C₁-C₄-alkoxy, C₁-C₄-haloalkoxy, C₁-C₄-alkylamino, di-C₁-C₄-alkylamino and C₁-C₄-alkylthio,
mono- to trinuclear aromatic ring system containing 6-14 carbon ring members, 5-membered hetaryl containing one to four nitrogen atoms or one to three nitrogen atoms and one sulfur or oxygen atom, or 6-membered hetaryl containing one to three nitrogen atoms,
it being possible for the (hetero)aromatic groups to be substituted by halogen, cyano, nitro, C₁-C₄-alkyl, C₁-C₄-haloalkyl, C₁-C₄-alkoxy, C₁-C₄-haloalkoxy, C₁-C₄-alkylamino, di-C₁-C₄-alkylamino and C₁-C₄-alkylthio and/or to have attached to them one or two of the following radicals: formyl or CRⁱⁱⁱ=NOR^{iv},
where Rⁱⁱⁱ is hydrogen or C₁-C₄-alkyl and R^{iv} is C₁-C₄-alkyl and phenyl C₁-C₄-alkyl,
or phenyl, pyridyl, pyrimidyl or triazinyl, it being possible for these groups to be substituted by halogen, cyano, nitro, C₁-C₄-alkyl, C₁-C₄-haloalkyl, C₁-C₄-alkoxy, C₁-C₄-haloalkoxy, C₁-C₄-alkylamino, di-C₁-C₄-alkylamino and C₁-C₄-alkylthio;
R^{a} is hydrogen, C₁-C₁₀-alkyl or C₁-C₁₀-haloalkyl;
W^{a} is oxygen, amino or C₁-C₁₀-alkylamino;
A² is hydrogen,
C₁-C₁₀-alkyl, C₂-C₁₀-alkenyl or C₂-C₁₀-alkynyl,
it being possible for these groups to be partially or fully halogenated and to have attached to them one of the following radicals: unsubstituted phenyl or phenyl which is substituted by halogen, cyano, nitro, C₁-C₄-alkyl, C₁-C₄-haloalkyl, C₁-C₄-alkoxy, C₁-C₄-haloalkoxy, C₁-C₄-alkylamino, di-C₁-C₄-alkylamino or C₁-C₄-alkylthio.

2. A compound of the formula I as claimed in claim 1 in which U is oxygen and V is oxygen or amino.

3. A compound of the formula I as claimed in claim 1 in which R¹ is methyl.

4. A compound of the formula I as claimed in claim 1 in which R² is methyl.

5. A process for the preparation of a compound I as claimed in claim 1 in which V is oxygen or sulfur, which comprises reacting a corresponding alcohol or a corresponding thiol of the formula IIa 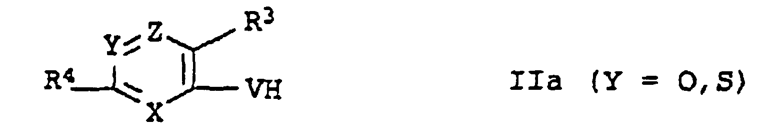 with a crotonate of the formula III 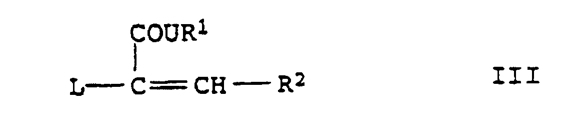 in which L is a nucleophilically exchangeable leaving group in a manner known per se in the presence of a base.

6. A process for the preparation of a compound I as claimed in claim 1 in which V is amino or C₁-C₁₀-alkylamino, which comprises reacting a corresponding amine of the formula IIb 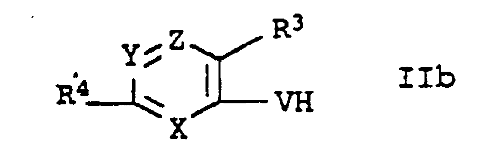 with an α-ketocarbonyl compound of the formula IV 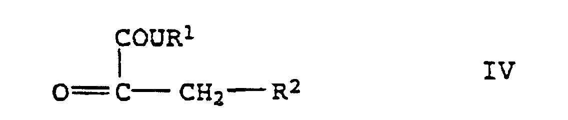 in a manner known per se in the presence of protonic acids.

7. A composition which is suitable for controlling animal pests or harmful fungi, comprising a solid or liquid carrier and a compound of the formula I as claimed in claim 1.

8. A method of controlling harmful fungi, which comprises treating the fungi or the materials, plants, soil or seed to be protected from fungal infection with an effective amount of a compound of the formula I as claimed in claim 1.

9. A method of controlling animal pests, which comprises treating the pests or the materials, plants, soil or seed to be protected from them with an effective amount of a compound of formula I as claimed in claim 1.

## Revendications

1. (Hétéro)aryloxy-, -thio- et -aminocrotonates de formule I 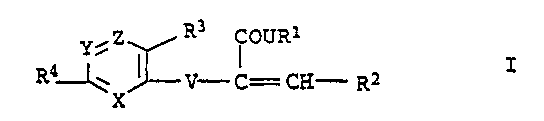 dans laquelle les symboles ont les significations suivantes :
U représente l'oxygène (-O-), le soufre (-S-) ou un groupe amino (-NH-) ;
V représente l'oxygène (-O-), le soufre (-S-), un groupe amino (-NH-) ou alkylamino en C₁-C₁₀ [-N(alkyle en C1-C10)-] ;
X, Y, Z représente, indépendamment les uns des autres =N- ou =CR³- ;
R¹, R² représentent, indépendamment l'un de l'autre, un groupe alkyle en C1-C4 ;
R³ représente l'hydrogène, un groupe cyano, nitro, un halogène, un groupe alkyle en C1-C4, halogénoalkyle en C1-C4, alcoxy en C1-C4, halogénoalcoxy en C1-C4, alkylthio en C1-C4 ou halogénoalkylthio en C1-C4 ;
R⁴ représente un groupe A¹-Y¹ ou un groupe A²-W^{a}-N=CR^{a}-;
Y¹ représente une liaison directe, l'oxygène, le soufre, un groupe amino ou alkylamino en C1-C10 ;
A¹ représente un groupe alkyle en C1-C10, alcényle en C2-C10 ou alcynyle en C2-C10, ces groupes pouvant être partiellement ou totalement halogénés et pouvant porter l'un des substituants suivants : phényle non substitué ou phényle lui-même substitué par des halogènes, des groupes cyano, nitro, alkyle en C1-C4, halogénoalkyle en C1-C4, alcoxy en C1-C4, halogénoalcoxy en C1-C4, alkylamino en C1-C4, di-(alkyle en C1-C4)amino et alkylthio en C1-C4,
un système cyclique aromatique mono- à tri-cyclique contenant 6 à 14 chaînons carbonés, un groupe hétéroaryle à 5 chaînons contenant un à quatre atomes d'azote ou un à trois atomes d'azote et un atome de soufre ou d'oxygène, ou un groupe hétéroaryle à six chaînons contenant un à trois atomes d'azote,
les groupes (hétéro)aromatiques pouvant porter des substituants halogéno, cyano, nitro, alkyle en C1-C4, halogénoalkyle en C1-C4, halogénoalcoxy en C1-C4, alkylamino en C1-C4, di-(alkyle en C1-C4)amino et alkylthio en C1-C4 et/ou pouvant porter un ou deux des groupes suivants : formyle ou Crⁱⁱⁱ=NOR^{iv},
Rⁱⁱⁱ représentant l'hydrogène ou un groupe alkyle en C1-C4 et R^{iv} un groupe alkyle en C1-C4 ou phényl-alkyle en C1-C4,
ou bien phényle, pyridyle, pyrimidyle ou triazinyle,
ces groupes pouvant porter des substituants halogéno, cyano, nitro, alkyle en C1-C4, halogénoalkyle en C1-C4, alcoxy en C1-C4, halogénoalcoxy en C1-C4, alkylamino en C1-C4, di-(alkyle en C1-C4)amino et alkylthio en C1-C4 ;
R^{a} représente l'hydrogène, un groupe alkyle en C1-C10 ou halogénoalkyle en C1-C10 ;
W^{a} représente l'hydrogène, un groupe amino ou alkylamino en C1-C10 ;
A² représente l'hydrogène,
un groupe alkyle en C1-C10, alcényle en C2-C10 ou alcynyle en C2-C10,
ces groupes pouvant être partiellement ou totalement halogénés et pouvant porter l'un des substituants suivants : phényle non substitué ou phényle portant des substituants halogéno, cyano, nitro, alkyle en C1-C4, halogénoalkyle en C1-C4, alcoxy en C1-C4, halogénoalcoxy en
C1-C4, alkylamino en C1-C4, di-(alkyle en C1-C4)amino ou alkylthio en C1-C4.

2. Composés de formule I selon la revendication 1, dans laquelle U représente l'oxygène et V l'oxygène ou un groupe amino.

3. Composés de formule I selon la revendication 1, dans laquelle R¹ représente un groupe méthyle.

4. Composés de formule I selon la revendication 1, dans laquelle R² représente un groupe méthyle.

5. Procédé de préparation des composés I de la revendication 1 dans laquelle V représente l'oxygène ou le soufre, caractérisé par le fait que l'on fait réagir un alcool correspondant ou un thiol correspondant de formule IIa 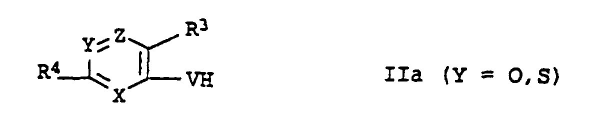 de manière connue en soi, en présence d'une base, avec un crotonate de formule III 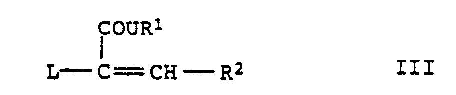 dans laquelle L représente un groupe éliminable par échange nucléophile.

6. Procédé de préparation des composés I de la revendication 1 dans laquelle V représente un groupe amino ou alkylamino en C1-C10, caractérisé par le fait que l'on fait réagir une amine correspondante de formule IIb 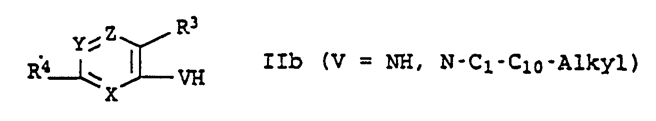 de manière connue en soi, en présence d'acides protoniques, avec un dérivé alpha-cétocarbonylé de formule IV

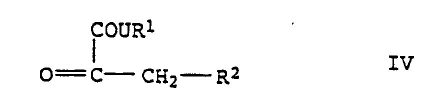

7. Produit approprié à l'utilisation pour la lutte contre les parasites animaux ou les mycètes nuisibles, contenant un véhicule solide ou liquide et un composé de formule générale I de la revendication 1.

8. Procédé pour combattre les mycètes nuisibles, caractérisé par le fait que l'on traite les mycètes ou les matériaux, végétaux, sols ou semences à protéger contre les mycètes par une quantité efficace d'un composé de formule générale I de la revendication 1.

9. Procédé pour combattre les parasites animaux, caractérisé par le fait que l'on traite les parasites ou les matériaux, végétaux, sols ou semences à protéger contre les parasites par une quantité efficace d'un composé de formule générale I de la revendication 1.
